# EUROPÄISCHE PATENTSCHRIFT

(11) **EP 3 268 942 B1**
(45) Veröffentlichungstag und Bekanntmachung des Hinweises auf die Patenterteilung: **19.06.2019**
(21) Anmeldenummer: 16700326.8
(22) Anmeldetag: 05.01.2016
(51) Int. Cl.: G08B 21/06

(54) **VERFAHREN UND VORRICHTUNG ZUM ERKENNEN EINES MÜDIGKEITS- UND/ODER SCHLAFZUSTANDES EINES FAHRERS EINES FAHRZEUGS**
METHOD AND DEVICE FOR DETECTING A TIREDNESS AND/OR SLEEPING STATE OF A DRIVER OF A VEHICLE
PROCÉDÉ ET DISPOSITIF D'IDENTIFICATION D'UN ÉTAT DE FATIGUE ET/OU DE SOMMEIL D'UN CONDUCTEUR D'UN VÉHICULE

(30) Priorität: 10.03.2015 DE 102015204247
(43) Veröffentlichungstag der Anmeldung: 17.01.2018
(73) Patentinhaber: Robert Bosch GmbH, 70442 Stuttgart (DE)
(72) Erfinder: WULF, Felix, 71636 Ludwigsburg (DE)
(86) Internationale Anmeldenummer: PCT/EP2016/050039
(87) Internationale Veröffentlichungsnummer: WO 2016/142074

(56) Entgegenhaltungen:
- DE-A1-102008 007 152
- US-A1- 2006 204 041
- US-A1- 2007 115 133
- US-A1- 2008 068 187

## Beschreibung

### Stand der Technik

Die vorliegende Erfindung bezieht sich auf ein Verfahren zum Erkennen eines Müdigkeits- und/oder Schlafzustandes eines Fahrers eines Fahrzeugs, auf eine entsprechende Vorrichtung sowie auf ein entsprechendes Computerprogramm.

Schläfrigkeit und Sekundenschlaf am Steuer führen häufig zu gefährlichen Situationen oder Unfällen. Schläfrigkeitserkenner geben beispielsweise eine Warnung aus, wenn der Fahrer einen gewissen Schläfrigkeitsgrenzwert überschreitet (= Kaffeetassen-Symbol).

Diese Warnung kann vom Fahrer jedoch ignoriert werden. Sekundenschlaf wird in vielen Schläfrigkeitsassistenten nicht als eigenständige Gefahr erkannt und somit findet keine rechtzeitige Warnung statt.

Schläfrigkeitserkennungssysteme schätzen die Müdigkeit des Fahrers indirekt aus dem Fahrverhalten. Sekundenschlaf wird als eigenständige Gefahrenquelle nicht beachtet.

Ebenfalls sind Systeme bekannt, die aus Daten einer Videokamera den momentanen Öffnungsgrad der Augen erkennen können (z. B. SmartEye, Facelab, etc..). Dies geschieht mit entsprechenden Bildverarbeitungsalgorithmen. Dabei wird jeweils für beide Augen ein Augenöffnungsniveau detektiert.

Die Druckschrift DE 10 2008 007 152 A1 offenbart ein Verfahren zur Parametrisierung eines Augenöffnungsgrades eines Fahrers eines Kraftfahrzeugs.

Die Druckschrift US 2008/0068187 A1 offenbart ein Verfahren und System zum Detektieren einer Bedienerwachheit.

### Offenbarung der Erfindung

Vor diesem Hintergrund werden mit dem hier vorgestellten Ansatz ein Verfahren zum Erkennen eines Müdigkeits- und/oder Schlafzustandes eines Fahrers eines Fahrzeugs, weiterhin eine Vorrichtung, die dieses Verfahren verwendet sowie schließlich ein entsprechendes Computerprogramm gemäß den Hauptansprüchen vorgestellt. Vorteilhafte Ausgestaltungen ergeben sich aus den jeweiligen Unteransprüchen und der nachfolgenden Beschreibung.

Der hier vorgestellte Ansatz schafft Verfahren zum Erkennen eines Müdigkeits- und/oder Schlafzustandes eines Fahrers eines Fahrzeugs, wobei das Verfahren die folgenden Schritte aufweist:
- Einlesen eines ersten Augenöffnungssignals und eines zweiten Augenöffnungssignals, wobei das erste Augenöffnungssignal einen Augenöffnungsgrad und/oder ein davon abgeleitetes Signal des linken Auges des Fahrers des Fahrzeugs und das zweite Augenöffnungssignal einen Augenöffnungsgrad und/oder ein davon abgeleitetes Signal des rechten Auges des Fahrers des Fahrzeugs repräsentiert;
- Erkennen einer Gültigkeit des ersten und zweiten Augenöffnungssignals, um das erste Augenöffnungssignal als gültig zu erkennen, wenn das erste Augenöffnungssignal einem ersten Kriterium entspricht und um das zweite Augenöffnungssignal als gültig zu erkennen, wenn das zweite Augenöffnungssignal einem zweiten Kriterium entspricht; und
- Bestimmen des Müdigkeits- und/oder Schlafzustandes des Fahrers des Fahrzeugs unter Verwendung des als gültig erkannten ersten Augenöffnungssignals und des als gültig erkannten zweiten

### Augenöffnungssignals.

Unter einem Augenöffnungsgrad kann beispielsweise ein Wert verstanden werden, zu wie viel Prozent das Augenlid das Auge bedeckt oder aber auch der absolute Abstand vom unteren und oberen Augenlid zueinander. Unter einem von dem Augenöffnungsgrad abgeleiteten Signal kann beispielsweise eine Augenöffnungsgeschwindigkeit, d. h. eine Geschwindigkeit der Bewegung des Augenlids oder eine Augenöffnungsbeschleunigung, d. h. eine Beschleunigung der Bewegung des Augenlids, verstanden werden. Beispielsweise kann dieses Signal durch die erste oder zweite Ableitung einer Bewegung des Augenlids bei der Bestimmung des Augenöffnungsgrads ermittelt werden. Dem ersten und/oder zweiten Augenöffnungssignal kann ferner ein Attribut zugeordnet werden, dass das betreffende Augenöffnungssignal als gültig gekennzeichnet. Ein solches als gültig gekennzeichnetes Augenöffnungssignal erfüllt ein entsprechendes Kriterium. Bei dem Bestimmen des Müdigkeits- und/oder Schlafzustandes des Fahrers des Fahrzeugs wird dann das erste und/oder zweite als gültig erkannte Augenöffnungssignal verwendet.

Der hier vorgeschlagene Ansatz basiert auf der Erkenntnis, dass eine sehr robuste Erkennung der Müdigkeit und/oder des Schlafzustandes des Fahrers dann möglich ist, wenn ein plausibilisiertes erstes und/oder zweites Augenöffnungssignal zu einer solchen Erkennung verwendet wird. Hierdurch kann ausgeschlossen oder zumindest weitgehend verhindert werden, dass durch Messfehler beim Erfassen eines Parameters des Auges oder des Augenlids ein falscher Wert für eine Müdigkeit und/oder einen Schlafzustand des Fahrers ermittelt wird. Der hier vorgeschlagene Ansatz bietet somit den Vorteil einer deutlich robusteren und zuverlässigeren Erkennung der Müdigkeit und/oder des Schlafzustands des Fahrers, wodurch falsche oder irrtümliche Warnungen an den Fahrer unterdrückt werden können. Dies führt zu einer erheblichen Steigerung der Akzeptanz der Müdigkeitserkennung bzw. Schlaferkennung des Fahrers durch den Fahrer, sodass eine entsprechende Warnung durch den Fahrer mit größerer Wahrscheinlichkeit ernst genommen wird.

Von Vorteil ist ferner eine Ausführungsform des hier vorgestellten Ansatzes, bei dem im Schritt des Erkennens das erste und/oder zweite Augenöffnungssignal als gültig erkannt wird, wenn ein die Augenöffnungsgeschwindigkeit des linken und/oder rechten Auges repräsentierender Wert einen Wert aufweist, der höchstens einen vorbestimmten Augenöffnungsgeschwindigkeitsschwellwert entspricht. Eine solche Ausführungsform des hier vorgestellten Ansatzes bietet den Vorteil, dass zwar bei einer Vibration der Kamera zur Erfassung des Augenöffnungsgrads eine sehr schnelle Bewegung des Augenlids erfasst wird, die jedoch nicht durch eine tatsächliche Bewegung des Augenlids, sondern durch die schnelle Vibrationsbewegung der Kamera verursacht ist, durch den Vergleich mit dem Augenöffnungsgeschwindigkeitsschwell(en)wert kann jedoch erkannt werden, dass diese solche schnelle Bewegung keine natürliche Bewegung eines Augenteils sein kann. Hierdurch wird eine Robustheit der Erkennung von Augenbewegungen bzw. Bewegungen von Teilen des Auges deutlich erhöht, die somit eine Fehleranfälligkeit bei einer entsprechenden Müdigkeitserkennung oder Schlaferkennung reduziert.

Günstig ist ferner eine Ausführungsform des hier vorgestellten Ansatzes, bei dem im Schritt des Einlesens ferner eine Kopfbewegungsgeschwindigkeit des Kopfes des Fahrers des Fahrzeugs eingelesen wird, wobei im Schritt des Erkennens das erste und/oder zweite Augenöffnungssignal als gültig erkannt wird, wenn die Kopfbewegungsgeschwindigkeit einen Wert aufweist, der höchstens einen vorbestimmten Kopfbewegungsgeschwindigkeitsschwellwert entspricht. Eine solche Ausführungsform des hier vorgestellten Ansatzes basiert auf der Erkenntnis, dass beispielsweise bei einer Fahrt über unebenes Gelände der Kopf des Fahrers stark schwankt, sodass die Erfassung des tatsächlichen Augenöffnungsgrads hierdurch möglicherweise fehlerhaft ist. Dadurch, dass das Augenöffnungssignal dann als gültig erkannt wird, wenn die Kopfbewegungsgeschwindigkeit höchstens einen vorbestimmten Schwellenwert erreicht, kann ein solcher möglicherweise fehlerbehafteter Wert des Augenöffnungssignals bei starken Kopfschwankungen für die Bestimmung der Müdigkeit bzw. des Schlafzustandes des Fahrers unterdrückt werden.

Von Vorteil ist ferner eine Ausführungsform des hier vorgestellten Ansatzes, bei dem im Schritt des Einlesens ferner eine Ausrichtung des Gesichts des Fahrers des Fahrzeugs einlesen wird, wobei im Schritt des Erkennens das erste und/oder zweite Augenöffnungssignal als gültig erkannt wird, wenn die Ausrichtung einen Wert aufweist, der innerhalb eines vorbestimmten Ausrichtungswinkelbereichs liegt. Eine solche Ausführungsform des hier vorgestellten Ansatzes bietet den Vorteil, dass mögliche Fehler bei der Erkennung des Augenöffnungsgrads durch ein starkes Verdrehen des Kopfes erkannt werden können, sodass für eine Müdigkeitserkennung bzw. Schlafzustandserkennung des Fahrers des Fahrzeugs ein Augenöffnungssignal zu einem Zeitpunkt eines solchen starken Verdrehens des Kopfes möglichst nicht verwendet werden sollte.

Besonders zuverlässig kann eine Müdigkeit bzw. ein Schlafzustand des Fahrers erkannt werden, wenn gemäß einer weiteren Ausführungsform des hier vorgestellten Ansatzes im Schritt des Bestimmens ein Gesamtaugenöffnungssignal unter Verwendung des ersten und/oder zweiten als gültig erkannten Augenöffnungssignals bestimmt wird, wobei der Müdigkeits- und/oder Schlafzustand des Fahrers des Fahrzeugs auf der Basis des Gesamtaugenöffnungssignals bestimmt wird. Eine solche Ausführungsform des hier vorgeschlagenen Ansatzes bietet den Vorteil, dass kleinere Fehler bei der Erkennung des Augenöffnungsgrads einem ersten Auge durch die Erfassung des Augenöffnungsgrads des zweiten Auges kompensiert werden können. Denkbar ist auch, dass eines der Augenöffnungssignale gar nicht für die Bestimmung der Müdigkeit und/oder des Schlafzustands des Fahrers verwendet wird, wenn eine Abweichung zwischen dem ersten und zweiten Augenöffnungssignal größer als ein vorbestimmter Schwellenwert ist, da in diesem Fall, der meist aus anatomischen Gründen bei den meisten Menschen nicht auftritt, von einer fehlerhaften Messung des Augenöffnungsgrads eines Auges auszugehen ist.

Gemäß einer weiteren Ausführungsform des hier vorgestellten Ansatzes kann im Schritt des Bestimmens ein als nicht als gültig erkanntes Augenöffnungssignal zum Bestimmen des Müdigkeits- und/oder Schlafzustandes des Fahrers des Fahrzeugs verworfen werden und/oder wobei ein als gültig erkanntes erstes Augenöffnungssignal und ein als gültig erkanntes zweites Augenöffnungssignal gemittelt werden, um den Müdigkeits- und/oder Schlafzustand des Fahrers zu bestimmen. Eine solche Ausführungsform des hier vorgestellten Ansatzes bietet den Vorteil einer robusten und zugleich technisch sehr einfachen und somit kostengünstigen Möglichkeit zur Bestimmung eines Augenöffnungsgrads oder eines davon abgeleiteten Signals und somit einer einfachen Möglichkeit zur Erkennung einer Müdigkeit und/oder eines Schlafzustands des Fahrers des Fahrzeugs.

Denkbar ist ferner eine Ausführungsform des hier vorgestellten Ansatzes, bei der im Schritt des Bestimmens ein Interpolieren eines ersten und/oder zweiten Augenöffnungssignals über eine Zeitspanne erfolgt, innerhalb der das erste und/oder zweite Augenöffnungssignal nicht als gültig erkannt wird. Auch eine solche Ausführungsform des hier vorgeschlagenen Ansatzes bietet den Vorteil einer technisch einfach umzusetzenden und zugleich robusten Variante zur Erkennung des Grades der Augenöffnung oder des davon abgeleiteten Signals für eines oder beide Augen.

Von Vorteil ist ferner eine Ausführungsform des hier vorgeschlagenen Ansatzes, bei dem im Schritt des Bestimmens ein Abschnitt eines als gültig erkannten ersten und/oder zweiten Augenöffnungssignals zum Bestimmen des Müdigkeits- und/oder Schlafzustandes des Fahrers des Fahrzeugs verworfen wird, wenn der Abschnitt kürzer als ein vorbestimmter Zeitdauerschwellwert ist. Besonders zuverlässig ist eine Ausführungsform des hier vorgestellten Ansatzes, bei dem ein Zeitabschnitt eines Augenöffnungssignals dann zur Erkennung der Müdigkeit und/oder des Schlafzustands des Fahrers des Fahrzeugs verwendet wird, wenn dieser Zeitabschnitt des Augenöffnungssignals länger als ein vorbestimmter Zeitdauerschwellenwert ist. Hierdurch wird sichergestellt, dass kurzzeitige Messfehler nicht zu einer falschen Warnung an den Fahrer führen, was die zukünftige Akzeptanz einer solchen Warnung durch den Fahrer reduzieren würde.

Um kurzzeitige Messfehler des Grades der Augenöffnung bei der Bestimmung der Müdigkeit und/oder des Schlafzustands des Fahrers des Fahrzeugs möglichst gut kompensieren zu können, kann gemäß einer weiteren Ausführungsform des hier vorgestellten Ansatzes auch im Schritt des Bestimmens das erste und/oder zweite als gültig erkannte Augenöffnungssignal und/oder das Gesamtaugenöffnungssignal geglättet werden.

Denkbar ist ferner eine Ausführungsform des hier vorgestellten Ansatzes, bei dem im Schritt des Erkennens das erste und/oder zweite Augenöffnungssignal einen Augenöffnungsgrad repräsentiert, der größer als ein vorbestimmter Augenöffnungsgradschwellwert ist. Auch eine solche Ausführungsform ermöglicht ein sehr robustes Erkennen einer Müdigkeit und/oder eines Schlafzustands, da aufgrund von physiologischen Gegebenheiten zumindest zeitweise das Auge soweit geöffnet ist, dass in einem kurzen Zeitabschnitt die Umgebung auch wahrgenommen werden kann. Ein ständiger Grad der Augenöffnung unterhalb des Augenöffnungsgradsschwellwerts ist somit physiologisch wenig wahrscheinlich und kann als Fehlmessung betrachtet werden, wodurch ein solches Signal nicht zur Bestimmung der Müdigkeit und/oder des Schlafzustands des Fahrers berücksichtigt werden braucht.

Der hier vorgestellte Ansatz schafft ferner eine Vorrichtung zum Erkennen eines Müdigkeits- und/oder Schlafzustandes eines Fahrers eines Fahrzeugs, wobei die Vorrichtung folgenden Merkmale aufweist:
- eine Schnittstelle zum Einlesen eines ersten Augenöffnungssignals und eines zweiten Augenöffnungssignals, wobei das erste Augenöffnungssignal einen Augenöffnungsgrad und/oder ein davon abgeleitetes Signal des linken Auges des Fahrers des Fahrzeugs und das zweite Augenöffnungssignal einen Augenöffnungsgrad und/oder ein davon abgeleitetes Signal des rechten Auges des Fahrers des Fahrzeugs repräsentiert;
- eine Einheit zum Erkennen einer Gültigkeit des ersten und/oder zweiten Augenöffnungssignals, um das erste Augenöffnungssignal als gültig zu erkennen, wenn das erste Augenöffnungssignal einem ersten Kriterium entspricht und/oder um das zweite Augenöffnungssignal als gültig zu erkennen, wenn das zweite Augenöffnungssignal einem zweiten Kriterium entspricht; und
- eine Einheit zum Bestimmen des Müdigkeits- und/oder Schlafzustandes des Fahrers des Fahrzeugs unter Verwendung des als gültig erkannten ersten Augenöffnungssignals und/oder des als gültig erkannten zweiten Augenöffnungssignals.

Der hier vorgestellte Ansatz schafft somit eine Vorrichtung, die ausgebildet ist, um die Schritte einer Variante eines hier vorgestellten Verfahrens in entsprechenden Einrichtungen durchzuführen, anzusteuern bzw. umzusetzen. Auch durch diese Ausführungsvariante der Erfindung in Form einer Vorrichtung kann die der Erfindung zugrunde liegende Aufgabe schnell und effizient gelöst werden.

Unter einer Vorrichtung kann vorliegend ein elektrisches Gerät verstanden werden, das Sensorsignale verarbeitet und in Abhängigkeit davon Steuer- und/oder Datensignale ausgibt. Die Vorrichtung kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen der Vorrichtung beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung, Umsetzung und/oder Ansteuerung der Schritte des Verfahrens nach einer der vorstehend beschriebenen Ausführungsformen verwendet wird, insbesondere wenn das Programmprodukt oder Programm auf einem Computer oder einer Vorrichtung ausgeführt wird.

Der hier vorgestellte Ansatz wird nachstehend anhand der beigefügten Zeichnungen beispielhaft näher erläutert. Es zeigen:
Fig. 1 ein Blockschaltbild eines Fahrzeugs, das ein Ausführungsbeispiel einer Vorrichtung zum Erkennen eines Müdigkeitszustands und/oder eines Schlafzustandes eines Fahrers des Fahrzeugs;
Fig. 2 ein Blockschaltbild eines Systems zur Erkennung eines Müdigkeitszustandes oder eines Schlafzustandes des Fahrers des Fahrzeugs mit einer Vorrichtung zum Erkennen des Müdigkeits - und/oder Schlafzustands des Fahrers eines Fahrzeugs gemäß einem Ausführungsbeispiel der vorliegenden Erfindung;
Fig. 3 ein detaillierteres Blockschaltbild eines Systems zur Erkennung eines Müdigkeitszustandes und/oder eines Schläfrigkeitszustandes eines Fahrers des Fahrzeugs; und
Fig. 4 ein Ablaufdiagramm eines Verfahrens gemäß einem Ausführungsbeispiel der vorliegenden Erfindung.

In der nachfolgenden Beschreibung günstiger Ausführungsbeispiele der vorliegenden Erfindung werden für die in den verschiedenen Figuren dargestellten und ähnlich wirkenden Elemente gleiche oder ähnliche Bezugszeichen verwendet, wobei auf eine wiederholte Beschreibung dieser Elemente verzichtet wird.

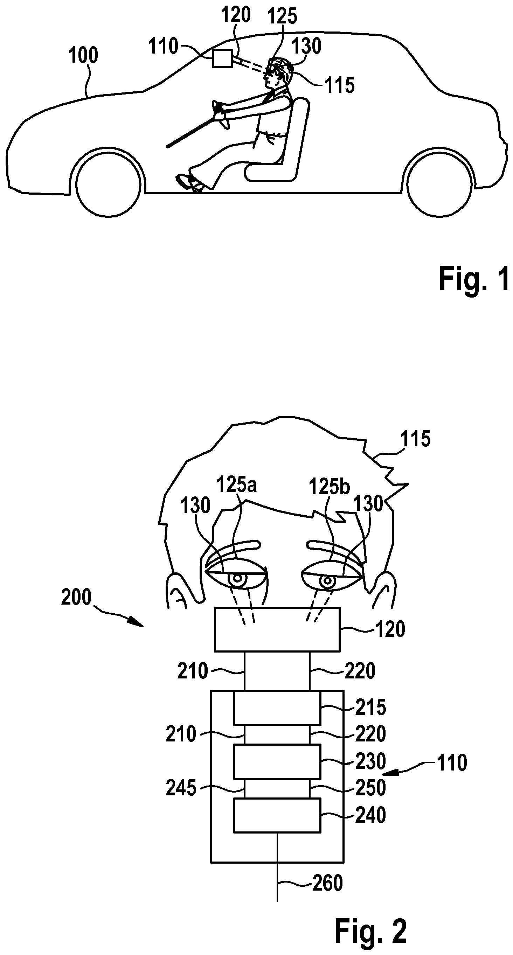

Fig. 1 zeigt ein Blockschaltbild eines Fahrzeugs 100, das ein Ausführungsbeispiel einer Vorrichtung 110 zum Erkennen eines Müdigkeitszustands und/oder eines Schlafzustandes eines Fahrers 115 des Fahrzeugs 100 aufweist. Die Vorrichtung 110 ist hierbei mit einer Kamera 120 verbunden, die die Augen 125 des Fahrers 115 erfasst, insbesondere eine Position eines Augenlids 130 des linken und/oder rechten Auges 125 erfasst und hieraus einen Grad der Augenöffnung des jeweiligen Auges 125 ermittelt.

Fig. 2 zeigt ein Blockschaltbild eines Systems 200 zur Erkennung eines Müdigkeitszustandes oder eines Schlafzustandes des Fahrers 115 des Fahrzeugs mit einer Vorrichtung 110 zum Erkennen des Müdigkeits - und/oder Schlafzustands des Fahrers 115 eines Fahrzeugs. Von der Kamera 120 wird dabei ein erstes Augenöffnungssignal 210 an eine Schnittstelle 215 zum Einlesen übersandt, das einen Augenöffnungsgrad oder ein davon abgeleitetes Signal des linken Auges 125a repräsentiert. Der Augenöffnungsgrad kann dabei ein Verhältnis des Lidschlusses des Augenlids 130 des linken Auges 125a zwischen einem vollständig geöffneten Lid 130 und einem vollständig geschlossenen Lid 130 repräsentieren. Beispielsweise kann das erste Augenöffnungssignal 210 auch ein von dem Augenöffnungsgrad abgeleitetes Signal wie beispielsweise eine Augenöffnungsgeschwindigkeit oder eine Augenöffnungsbeschleunigung des linken Auges 125a repräsentieren, beispielsweise wenn durch die Kamera 120 ein zeitlicher Verlauf der Positionen des Augenlids 130 erfasst und entsprechend differenziert wird.

Analog wird von der Kamera 120 ein zweites Augenöffnungssignal 220 an die Schnittstelle 215 zum Einlesen übermittelt, das analog zum ersten Augenöffnungssignal 310 nun jedoch einen Augenöffnungsgrad oder ein davon abgeleitetes Signal des rechten Auges 125b repräsentiert. Der Augenöffnungsgrad kann hierbei ebenfalls ein Verhältnis des Lidschlusses des Augenlids 130 des rechten Auges 125b zwischen einem vollständig geöffneten Lid 130 und einem vollständig geschlossenen Lid 130 repräsentieren. Beispielsweise kann das zweite Augenöffnungssignal 220 ebenfalls auch ein von dem Augenöffnungsgrad abgeleitetes Signal wie beispielsweise eine Augenöffnungsgeschwindigkeit oder eine Augenöffnungsbeschleunigung des rechten Auges 125b repräsentieren, beispielsweise wenn durch die Kamera 120 ein zeitlicher Verlauf der Positionen des Augenlids 130 erfasst und entsprechend differenziert wird.

Die Vorrichtung 110 zum Erkennen des Müdigkeitszustands und/oder Schlafzustandes des Fahrers 115 weist ferner eine Einheit 230 zum Erkennen auf, der von der Schnittstelle 215 das jeweils eingelesene erste Augenöffnungssignal 210 und zweite Augenöffnungssignal 220 übermittelt wird. In der Einheit 230 zum Erkennen wird das erste Augenöffnungssignal 210 als gültig erkannt, wenn es einem nachfolgend noch näher beschriebenen ersten Kriterium entspricht. In diesem Fall wird einer Einheit 240 zum Bestimmen ein als gültig erkanntes erstes Augenöffnungssignal 245 übermittelt. In der Einheit 230 zum Erkennen wird ferner auch das zweite Augenöffnungssignal 220 als gültig erkannt, wenn es auf analoge Weise einem ebenfalls nachfolgend noch näher beschriebenen zweiten Kriterium entspricht. In diesem Fall wird der Einheit 240 zum Bestimmen ein als gültig erkanntes zweites Augenöffnungssignal 250 übermittelt.

Das erste Kriterium kann dabei zum zweiten Kriterium identisch sein. Denkbar ist jedoch auch, dass das erste Kriterium unterschiedlich vom zweiten Kriterium ist, beispielsweise wenn für das linke und rechte Auge unterschiedliche anatomische Verhältnisse vorliegen.

Von der Einheit 240 zum Bestimmen wird der Müdigkeitszustand und/oder Schlafzustand des Fahrers 115 des Fahrzeugs 100 dann unter Verwendung des als gültig erkannten ersten Augenöffnungssignals 245 und/oder des als gültig erkannten zweiten Augenöffnungssignals 250 bestimmt und ein entsprechendes Signal 260 ausgegeben. Dieses Signal 260 repräsentiert dann den Möglichkeitszustand und/oder Schlafzustand des Fahrers 115 und kann beispielsweise zur Ausgabe einer Warnmeldung im Fahrzeug 100 verwendet werden.

Der hier vorgestellte Ansatz stellt eine Verbesserung der Erkennungsgüte des Augenöffnungsniveaus für Müdigkeitserkennungs-Systeme und Sekundenschlaferkennungs-Systeme dar. Unter der Annahme, dass sich während der Fahrt beide Augen 125 nur zusammen (und nicht getrennt voneinander) bewegen, ist es das Ziel der Erfindung, die Robustheit und Erkennungsgüte des Augenöffnungsniveaus zu verbessern.

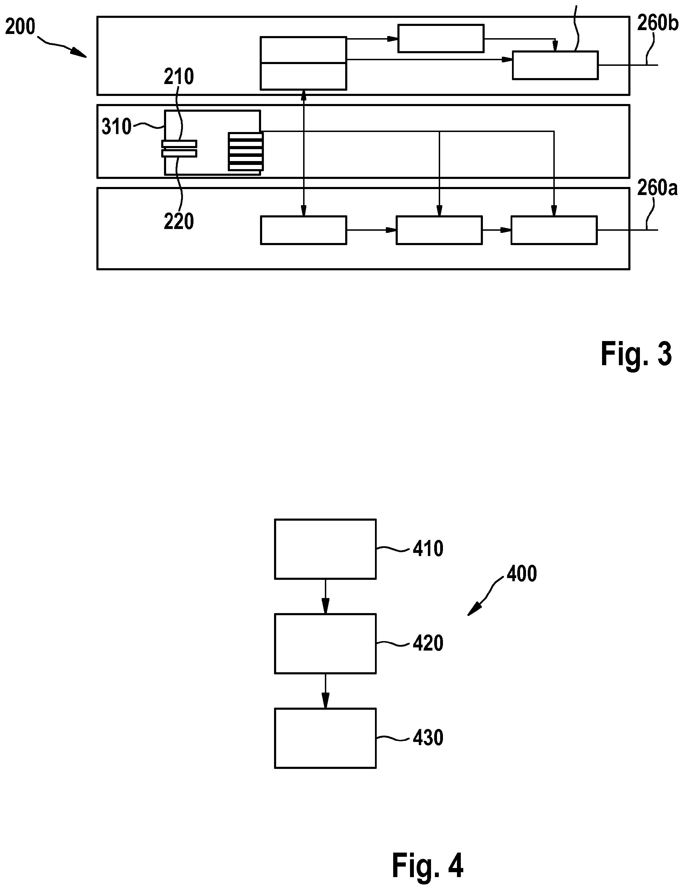

Dabei werden auf die Einzel-Signale von linkem 125a und rechtem 125b Auge zurückgegriffen und ein gemeinsames Augenöffnungsniveau und eventuell weitere Parameter berechnet. Der hier vorgestellte Ansatz, der auch als Eye Closure Preprocessing (ECP) bezeichnet werden kann, gliedert sich in ein Gesamt-System zur Erkennung von Schläfrigkeit und/oder Sekundenschlaf ein, wie es in der Fig.3 als Blockschaltbild näher dargestellt ist.

Fig. 3 zeigt ein detaillierteres Blockschaltbild eines Systems 200 zur Erkennung eines Müdigkeitszustandes und/oder eines Schläfrigkeitszustandes eines Fahrers 115 des Fahrzeugs. Hierbei wird zunächst in einer Einheit 310 eine Augenschluss-Vorverarbeitung (ECP) durchgeführt, bei der beispielsweise der Augenöffnungsgrad des linken und rechten Auges eingelesen wird, wobei entsprechende Ausgangssignale wie beispielsweise ein Tiefpass-gefilterter Augenöffnungsgrad, eine Augenöffnungsbeschleunigung, eine Augenöffnungsgeschwindigkeit und/oder eine Gültigkeit des betreffenden Augenöffnungssignals erfasst wird. Insofern entspricht die Einheit 310 aus Fig. 3 den Einheiten 215 und 230 der Vorrichtung 110 entsprechend der Fig. 2.

In einer ersten Variante der Einheit 240 zum Bestimmen kann nun eine Mikroschlaf-Erkennung durchgeführt werden, in welcher auf der Basis der von der Einheit 310 gelieferten Signale erkannt wird, ob tatsächlich ein Mikroschlaf-Verhalten des Fahrers 115 vorliegt und dieses in einem ersten Teilsignal 260 a ausgegeben.

In einer zweiten Variante der Einheit 240 zum Bestimmen kann nun eine Müdigkeitsklassifikation des Fahrers 115 erfolgen. Hierzu wird eine Klassifizierung der Müdigkeit des Fahrers vorgenommen und in einem zweiten Teilsignal 260 b eine entsprechende Information ausgegeben.

Mit Bezug zur Fig. 2 wurde bereits ein erstes und zweites Kriterium genannt, das/die verwendet werden soll/sollen, um das erste bzw. zweite Augenöffnungssignal als gültig zu erkennen. Im Nachfolgenden werden einige Möglichkeiten aufgezeigt, die als ein solches Kriterium verwendet werden können. Hierbei wird zur einfacheren Darstellung lediglich das Kriterium selbst vorgestellt, das als erstes und/oder zweites Kriterium in der Einheit 230 zum Erkennen verwendet werden kann. Vorab sein angemerkt, dass in den unterschiedlichen Ausführungsbeispielen der Einheit 230 zum Erkennen unterschiedliche Varianten für das erste und zweite Kriterium verwendet werden können. Denkbar ist jedoch auch, dass identische Kriterien als erstes und zweites Kriterium verwendet werden.

Folgende Gültigkeitskriterien können als erstes und/oder zweites Kriterium (beispielsweise jeweils getrennt für das rechte und linke Auge) ausgewertet werden. Diese Kriterien können im resultierenden Produkt jeweils zusammen aber auch getrennt verwendet werden:
- Verfügbarkeit: Das Signal (hier das jeweilige Augenöffnungssignal 210 bzw. 220) sollte überhaupt verfügbar sein.
- Qualität: Das von der Bildverarbeitung (hier der Kamera 210 bzw. der Einheit 230 zum Erkennen) gemeldete Qualitätssignal 210, 220, 245 bzw. 250 für das Erkennen des jeweiliges Auges sollte einen Augenöffnungsgrad repräsentieren, der oberhalb von einem bestimmten Grenzwert (z. B. > 0.2) liegt.
- Augenöffnungsgeschwindigkeit: In manchen Fällen springt das im Bild erkannte Lid 130 zwischen zwei Verläufen hin- und her. Dieses Verhalten kann im (Augenöffnungssignal 210 bzw. 220) relativ einfach durch ein Hin- und Her-Springen des Signalwerts erkannt werden. Dabei ist die resultierende gemessene Augengeschwindigkeit größer als die physiologisch maximale Augenlidgeschwindigkeit. Sobald diese Augenlidgeschwindigkeit somit einen bestimmten Grenzwert überschreitet, werden die dazugehörigen Bereiche des Augenöffnungsgrades bzw. -signals des jeweiligen Auges als ungültig markiert. Anderenfalls, also wenn die Augenlidgeschwindigkeit somit einen bestimmten Grenzwert unterschreitet, werden die die dazugehörigen Bereiche des Augenöffnungsgrades bzw. - signals des jeweiligen Auges als gültig markiert.
- Kopfpose: Das resultierende Augenschlusssignal bzw. Augenöffnungssignal 210 bzw. 220 ist nur innerhalb von bestimmten Kopfposen (z. B. Kopfdrehung weniger als 45° nach rechts oder links) verwendbar. Diese können anhand der Ausrichtung des Gesichts des Fahrers 115 erkannt werden und die bestimmten (Winkel-) Bereiche als ungültig bzw. gültig markiert werden.
- Kopfgeschwindigkeit: Gerade bei hoher Dynamik der Kopfbewegung (z. B. bei einer Fahrt über eine Fahrbahn mit einer Vielzahl von Schlaglöchern) kann die Bildverarbeitung in manchen Fällen das Augenöffnungssignal 210 bzw. 220 nicht gut genug der tatsächlichen Position der Augen 125 des Fahrers 115 nachführen, sodass möglicherweise fehlerhafte Ergebnisse bei der Auswertung des Augenöffnungsgrades resultieren, die auf die nicht ausreichend schnelle Nachführung des Bereichs der Augen durch die Auswertungseinheiten im Fahrzeug 100 zurückzuführen sind. Hier bietet sich die Verwendung von einem Grenzwert für die Geschwindigkeit des Kopfes an. Sobald diese einen bestimmten Grenzwert überschreitet, werden die zugehörigen Bereiche des Augenöffnungssignals als ungültig markiert, bzw. andersherum werden diejenigen (beispielsweise zeitlichen) Bereiche des Augenöffnungssignals als gültig erkannt, in denen die Bewegungsgeschwindigkeit des Kopfes des Fahrers 115 kleiner als der Grenzwert ist.

Ferner kann auch ein Zusammenführen der gültigen Augenöffnungssignale von beiden Augen zu einem (Gesamt-) Signal erfolgen. Das resultierende Gesamt-) Augenöffnungssignal kann dann in Abhängigkeit von der Gültigkeit der Augenöffnungssignale von linkem und rechtem Auge beispielsweise folgendermaßen bestimmt werden:
- Wenn beide (Augenöffnungs-) Signale gültig sind, wird ein (möglicherweise gewichteter) Mittelwert gebildet. Als Gewichtungsfaktoren bietet sich hier z. B. das Qualitäts-Signal der Bildverarbeitung an. Hierzu kann dem als gültig erkannten ersten und/oder zweiten Augenöffnungssignal ein Qualitätsparameter zugeordnet werden, den Grad der Zuverlässigkeit oder Gültigkeit des Wertes des Augenöffnungssignals repräsentiert und der dann zur Gewichtung bei der Bildung des Mittelwertes verwendet werden kann.
- Wenn nur ein (Augenöffnungs-) Signal gültig ist (linkes oder rechtes Auge), wird (nur) dieses (Augenöffnungs-) Signal verwendet.
- Wenn kein (Augenöffnungs-) Signal gültig ist, wird auch das resultierende gemittelte Augenschlusssignal bzw. ein entsprechendes Eingangssignal bzw. die entsprechenden Eingangssignale 245 bzw. 250 für die Einheit 240 zum Bestimmen als ungültig oder nicht als gültig deklariert.

Für das so berechnete Gesamt-Signal und/oder die jeweiligen Augenöffnungssignale 210 bzw. 220 und/oder die als gültig erkannten jeweiligen Augenöffnungssignale 245 bzw. 250 sind folgende weiteren BearbeitungsSchritte denkbar:
- Interpolieren von kurzen ungültigen Bereichen (z. B. t_ungültig < 50ms).
- Entfernen von kurzen gültigen Bereichen (z. B. t_gültig < 1.5 s).
- Glättung des Signals (z. B. mit einem Savitzky-Golay Filter)

Mit dem berechneten und geglätteten Augenöffnungsgrad können im Rahmen des Preprocessings weitere Signale berechnet werden:
- Geschwindigkeit der Augenlider 130 durch Ableitung des AugenöffnungsSignals und/oder Ableitung eines zeitlichen Verhaltens des Augenöffnungsgrades
- Beschleunigung der Augenlider 130 durch Ableitung des GeschwindigkeitsSignals und/oder Ableitung (insbesondere der zweiten Ableitung) eines zeitlichen Verhaltens des Augenöffnungsgrades
- Momentanes Augenöffnungsniveau (= Augenöffnung ohne Berücksichtigung von neu Blinzlern) z. B. mit Hilfe von einem Savitzky-Golay Filter

Die so berechneten Signale 245 bz. 250 können für die Detektion von Sekundenschlaf und Schläfrigkeit verwendet werden.

Fig. 4 zeigt ein Ablaufdiagramm eines Verfahrens 400 zum Erkennen eines Müdigkeits- und/oder Schlafzustandes eines Fahrers eines Fahrzeugs. Das Verfahren 400 umfasst einen Schritt 410 des Einlesens eines ersten Augenöffnungssignals und eines zweiten Augenöffnungssignals, wobei das erste Augenöffnungssignal einen Augenöffnungsgrad und/oder ein davon abgeleitetes Signal des linken Auges des Fahrers des Fahrzeugs und das zweite Augenöffnungssignal einen Augenöffnungsgrad und/oder ein davon abgeleitetes Signal des rechten Auges des Fahrers des Fahrzeugs repräsentiert. Ferner umfasst das Verfahren 400 einen Schritt 420 des Erkennens einer Gültigkeit des ersten und/oder zweiten Augenöffnungssignals, um das erste Augenöffnungssignal als gültig zu erkennen, wenn das erste Augenöffnungssignal einem ersten Kriterium entspricht und/oder um das zweite Augenöffnungssignal als gültig zu erkennen, wenn das zweite Augenöffnungssignal einem zweiten Kriterium entspricht. Schließlich umfasst das Verfahren 400 einen Schritt 430 des Bestimmens des Müdigkeits- und/oder Schlafzustandes des Fahrers des Fahrzeugs unter Verwendung des als gültig erkannten ersten Augenöffnungssignals und/oder des als gültig erkannten zweiten Augenöffnungssignals.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Verfahren (400) zum Erkennen (420) eines Müdigkeits- und/oder Schlafzustandes eines Fahrers (115) eines Fahrzeugs (100), wobei das Verfahren (400) die folgenden Schritte aufweist:
- Einlesen (410) eines ersten Augenöffnungssignals (210) und eines zweiten Augenöffnungssignals (220), wobei das erste Augenöffnungssignal (210) einen Augenöffnungsgrad und/oder ein davon abgeleitetes Signal des linken Auges (125a) des Fahrers (115) des Fahrzeugs (100) und das zweite Augenöffnungssignal (220) einen Augenöffnungsgrad und/oder ein davon abgeleitetes Signal des rechten Auges (125b) des Fahrers (115) des Fahrzeugs (100) repräsentiert;
- Erkennen (420) einer Gültigkeit des ersten und zweiten Augenöffnungssignals (210, 220), um das erste Augenöffnungssignal (245) als gültig zu erkennen, wenn das erste Augenöffnungssignal (210) einem ersten Kriterium entspricht und um das zweite Augenöffnungssignal (250) als gültig zu erkennen, wenn das zweite Augenöffnungssignal (220) einem zweiten Kriterium entspricht; und
- Bestimmen (430) des Müdigkeits- und/oder Schlafzustandes des Fahrers (115) des Fahrzeugs (100) unter Verwendung des als gültig erkannten ersten Augenöffnungssignals (245) und des als gültig erkannten zweiten Augenöffnungssignals (250).

2. Verfahren (400) gemäß Anspruch 1, **dadurch gekennzeichnet, dass** im Schritt des Erkennens (420) das erste und/oder zweite Augenöffnungssignal (210, 220) als gültig erkannt wird, wenn ein die Augenöffnungsgeschwindigkeit des linken (125a) und/oder rechten (125b) Auges repräsentierender Wert einen Wert aufweist, der einen vorbestimmten Augenöffnungsgeschwindigkeitsschwellwert nicht überschreitet.

3. Verfahren (400) gemäß einem der vorangegangenen Ansprüche, **dadurch gekennzeichnet, dass** im Schritt des Einlesens (410) ferner eine Kopfbewegungsgeschwindigkeit des Kopfes des Fahrers (115) des Fahrzeugs (100) eingelesen wird, wobei im Schritt des Erkennens (420) das erste und/oder zweite Augenöffnungssignal (210, 220) als gültig erkannt wird, wenn die Kopfbewegungsgeschwindigkeit einen Wert aufweist, der höchstens einem vorbestimmten Kopfbewegungsgeschwindigkeitsschwellwert entspricht.

4. Verfahren (400) gemäß einem der vorangegangenen Ansprüche, **dadurch gekennzeichnet, dass** im Schritt des Einlesens (410) ferner eine Ausrichtung des Gesichts des Fahrers (115) des Fahrzeugs (100) eingelesen wird, wobei im Schritt des Erkennens (420) das erste und/oder zweite Augenöffnungssignal (210, 220) als gültig erkannt wird, wenn die Ausrichtung einen Wert aufweist, der innerhalb eines vorbestimmten Ausrichtungswinkelbereichs liegt.

5. Verfahren (400) gemäß einem der vorangegangenen Ansprüche, **dadurch gekennzeichnet, dass** im Schritt des Bestimmens (430) ein Gesamtaugenöffnungssignal unter Verwendung des ersten und/oder zweiten als gültig erkannten Augenöffnungssignals (245, 250) bestimmt wird, wobei der Müdigkeits- und/oder Schlafzustand des Fahrers (115) des Fahrzeugs (100) auf der Basis des Gesamtaugenöffnungssignals bestimmt wird.

6. Verfahren (400) gemäß einem der vorangegangenen Ansprüche, **dadurch gekennzeichnet, dass** im Schritt des Bestimmens (430) ein nicht als gültig erkanntes Augenöffnungssignal (245, 250) zum Bestimmen (430) des Müdigkeits- und/oder Schlafzustandes des Fahrers (115) des Fahrzeugs (100) verworfen wird und/oder wobei ein als gültig erkanntes erstes Augenöffnungssignal (245) und ein als gültig erkanntes zweites Augenöffnungssignal (250) gemittelt wird, um den Müdigkeits- und/oder Schlafzustand des Fahrers (115) zu bestimmen.

7. Verfahren (400) gemäß einem der vorangegangenen Ansprüche, **dadurch gekennzeichnet, dass** im Schritt des Bestimmens (430) ein Interpolieren eines ersten und/oder zweiten Augenöffnungssignals (210, 220) und/oder des als gültig erkannten ersten und/oder zweiten Augenöffnungssignals (245, 250) über eine Zeitspanne erfolgt, innerhalb der das erste und/oder zweite Augenöffnungssignal (210, 220) und/oder das als gültig erkannte erste und/oder zweite Augenöffnungssignal (245, 250) nicht als gültig erkannt wird.

8. Verfahren (400) gemäß einem der vorangegangenen Ansprüche, **dadurch gekennzeichnet, dass** im Schritt des Bestimmens (430) ein Abschnitt eines als gültig erkannten ersten und/oder zweiten Augenöffnungssignals (245, 250) zum Bestimmen (430) des Müdigkeits- und/oder Schlafzustandes des Fahrers (115) des Fahrzeugs (100) verworfen wird, wenn der Abschnitt kürzer als ein vorbestimmter Zeitdauerschwellwert ist.

9. Verfahren (400) gemäß einem der vorangegangenen Ansprüche, **dadurch gekennzeichnet, dass** im Schritt des Bestimmens (430) das erste und/oder zweite als gültig erkannte Augenöffnungssignal (245, 250) und/oder das Gesamtaugenöffnungssignal geglättet wird.

10. Verfahren (400) gemäß einem der vorangegangenen Ansprüche, **dadurch gekennzeichnet, dass** im Schritt des Erkennens (420) das erste und/oder zweite Augenöffnungssignal (210, 220) und/oder das als gültig erkannte erste und/oder zweite Augenöffnungssignal (245, 250) einen Augenöffnungsgrad repräsentiert, der größer als ein vorbestimmter Augenöffnungsgradschwellwert ist.

11. Vorrichtung (110), die ausgebildet ist, um alle Schritte eines Verfahrens (400) gemäß einem der vorangegangenen Ansprüche in entsprechenden Einheiten (215, 230, 240) durchzuführen, umzusetzen und/oder anzusteuern.

12. Computerprogramm, das dazu eingerichtet ist, alle Schritte eines Verfahrens (400) gemäß einem der vorangegangenen Ansprüche 1 bis 10 durchzuführen, umzusetzen und/oder anzusteuern..

13. Maschinenlesbares Speichermedium mit einem darauf gespeicherten Computerprogramm nach Anspruch 12.

## Claims

1. Method (400) for recognizing (420) a state of tiredness and/or a state of sleep of a driver (115) of a vehicle (100), the method (400) having the following steps:
- reading in (410) a first eye opening signal (210) and a second eye opening signal (220), the first eye opening signal (210) representing a degree of eye opening, and/or a signal derived therefrom, of the left eye (125a) of the driver (115) of the vehicle (100), and the second eye opening signal (220) representing a degree of eye opening, and/or a signal derived therefrom, of the right eye (125b) of the driver (115) of the vehicle (100);
- recognizing (420) a validity of the first and second eye opening signals (210, 220) in order to recognize the first eye opening signal (245) as valid if the first eye opening signal (210) corresponds to a first criterion and in order to recognize the second eye opening signal (250) as valid if the second eye opening signal (220) corresponds to a second criterion; and
- determining (430) the state of tiredness and/or the state of sleep of the driver (115) of the vehicle (100) using the first eye opening signal (245) recognized as valid and the second eye opening signal (250) recognized as valid.

2. Method (400) according to Claim 1, **characterized in that,** in the recognizing step (420), the first and/or second eye opening signal (210, 220) is recognized as valid if a value representing the eye opening speed of the left eye (125a) and/or right eye (125b) has a value that does not exceed a predetermined eye opening speed threshold value.

3. Method (400) according to one of the preceding claims, **characterized in that,** in the reading-in step (410), a head movement speed of the head of the driver (115) of the vehicle (100) is also read in, the first and/or second eye opening signal (210, 220) being recognized as valid in the recognizing step (420) if the head movement speed has a value that corresponds at most to a predetermined head movement speed threshold value.

4. Method (400) according to one of the preceding claims, **characterized in that,** in the reading-in step (410), an orientation of the face of the driver (115) of the vehicle (100) is also read in, the first and/or second eye opening signal (210, 220) being recognized as valid in the recognizing step (420) if the orientation has a value that is within a predetermined orientation angular range.

5. Method (400) according to one of the preceding claims, **characterized in that,** in the determining step (430), an overall eye opening signal is determined using the first and/or second eye opening signal (245, 250) recognized as valid, the state of tiredness and/or the state of sleep of the driver (115) of the vehicle (100) being determined on the basis of the overall eye opening signal.

6. Method (400) according to one of the preceding claims, **characterized in that,** in the determining step (430), an eye opening signal (245, 250) not recognized as valid is rejected for determining (430) the state of tiredness and/or the state of sleep of the driver (115) of the vehicle (100), and/or **in that** a first eye opening signal (245) recognized as valid and a second eye opening signal (250) recognized as valid are averaged in order to determine the state of tiredness and/or the state of sleep of the driver (115).

7. Method (400) according to one of the preceding claims, **characterized in that,** in the determining step (430), a first and/or second eye opening signal (210, 220) and/or the first and/or second eye opening signal (245, 250) recognized as valid is/are interpolated over a time span within which the first and/or second eye opening signal (210, 220) and/or the first and/or second eye opening signal (245, 250) recognized as valid is/are not recognized as valid.

8. Method (400) according to one of the preceding claims, **characterized in that,** in the determining step (430), a segment of a first and/or second eye opening signal (245, 250) recognized as valid for determining (430) the state of tiredness and/or the state of sleep of the driver (115) of the vehicle (100) is rejected if the segment is shorter than a predetermined time duration threshold value.

9. Method (400) according to one of the preceding claims, **characterized in that,** in the determining step (430), the first and/or second eye opening signal (245, 250) recognized as valid and/or the overall eye opening signal is/are smoothed.

10. Method (400) according to one of the preceding claims, **characterized in that,** in the recognizing step (420), the first and/or second eye opening signal (210, 220) and/or the first and/or second eye opening signal (245, 250) recognized as valid represent(s) a degree of eye opening that is greater than a predetermined eye opening degree threshold value.

11. Apparatus (110) which is designed to carry out, implement and/or control all steps of a method (400) according to one of the preceding claims in corresponding units (215, 230, 240).

12. Computer program which is set up to carry out, implement and/or control all steps of a method (400) according to one of the preceding Claims 1 to 10.

13. Machine-readable storage medium having a computer program according to Claim 12 stored thereon.

## Revendications

1. Procédé (400) pour reconnaître (420) un état de fatigue et/ou de sommeil d'un conducteur (115) d'un véhicule (100), dans lequel le procédé (400) présente les étapes suivantes :
- la lecture (410) d'un premier signal d'ouverture d'oeil (210) et d'un deuxième signal d'ouverture d'oeil (220), dans lequel le premier signal d'ouverture d'oeil (210) représente un degré d'ouverture d'oeil et/ou un signal qui en est dérivé de l'oeil gauche (125a) du conducteur (115) du véhicule (100) et le deuxième signal d'ouverture d'oeil (220) représente un degré d'ouverture d'oeil et/ou un signal qui en est dérivé de l'oeil droit (125b) du conducteur (115) du véhicule (100) ;
- la reconnaissance (420) d'une validité du premier et deuxième signal d'ouverture d'oeil (210, 220) pour reconnaître le premier signal d'ouverture d'oeil (245) comme étant valide lorsque le premier signal d'ouverture d'oeil (210) correspond à un premier critère et pour reconnaître le deuxième signal d'ouverture d'oeil (250) comme étant valide lorsque le deuxième signal d'ouverture d'oeil (220) correspond à un deuxième critère ; et
- la détermination (430) de l'état de fatigue et/ou de sommeil du conducteur (115) du véhicule (100) en utilisant le premier signal d'ouverture d'oeil (245) reconnu comme étant valide et le deuxième signal d'ouverture d'oeil (250) reconnu comme étant valide.

2. Procédé (400) selon la revendication 1, **caractérisé en ce qu'**à l'étape de reconnaissance (420), le premier et/ou deuxième signal d'ouverture d'oeil (210, 220) est reconnu comme étant valide si une valeur représentant la vitesse d'ouverture d'oeil de l'oeil gauche (125a) et/ou de l'oeil droit (125b) présente une valeur qui ne dépasse pas une valeur de seuil de vitesse d'ouverture d'oeil prédéfinie.

3. Procédé (400) selon l'une des revendications précédentes, **caractérisé en ce qu'**à l'étape de lecture (410), on lit en outre une vitesse de mouvement de tête de la tête du conducteur (115) du véhicule (100), dans lequel, à l'étape de reconnaissance (420), le premier et/ou deuxième signal d'ouverture d'oeil (210, 220) est reconnu comme étant valide si la vitesse de mouvement de tête présente une valeur, laquelle correspond au maximum à une valeur de seuil de vitesse de mouvement de tête prédéfinie.

4. Procédé (400) selon l'une des revendications précédentes, **caractérisé en ce qu'**à l'étape de lecture (410), on lit en outre une orientation du visage du conducteur (115) du véhicule (100), dans lequel, à l'étape de reconnaissance (420), le premier et/ou deuxième signal d'ouverture d'oeil (210, 220) est reconnu comme étant valide si l'orientation présente une valeur, laquelle se situe dans les limites d'une plage d'angle d'orientation prédéfinie.

5. Procédé (400) selon l'une des revendications précédentes, **caractérisé en ce qu'**à l'étape de détermination (430), on détermine un signal d'ouverture d'oeil global en utilisant le premier et/ou deuxième signal d'ouverture d'oeil (245, 250) reconnu comme étant valide, dans lequel l'état de fatigue et/ou de sommeil du conducteur (115) du véhicule (100) est déterminé sur la base du signal d'ouverture d'oeil global.

6. Procédé (400) selon l'une des revendications précédentes, **caractérisé en ce qu'**à l'étape de détermination (430), un signal d'ouverture d'oeil (245, 250) reconnu comme étant non valide pour la détermination (430) de l'état de fatigue et/ou sommeil du conducteur (115) du véhicule (100) est rejeté et/ou dans lequel on calcule la moyenne d'un premier signal d'ouverture d'oeil (245) reconnu comme étant valide et d'un deuxième signal d'ouverture d'oeil (250) reconnu comme étant valide pour déterminer l'état de fatigue et/ou de sommeil du conducteur (115).

7. Procédé (400) selon l'une des revendications précédentes, **caractérisé en ce qu'**à l'étape de détermination (430), une interpolation d'un premier et/ou deuxième signal d'ouverture d'oeil (210, 220) et/ou du premier et/ou deuxième signal d'ouverture d'oeil (245, 250) reconnu comme étant valide a lieu sur un intervalle de temps pendant lequel le premier et/ou deuxième signal d'ouverture d'oeil (210, 220) et/ou le premier et/ou deuxième signal d'ouverture d'oeil (245, 250) reconnu comme étant valide n'est pas reconnu comme étant valide.

8. Procédé (400) selon l'une des revendications précédentes, **caractérisé en ce qu'**à l'étape de détermination (430), un segment d'un premier et/ou deuxième signal d'ouverture d'oeil (245, 250) reconnu comme étant valide pour la détermination (430) de l'état de fatigue et/ou de sommeil du conducteur (115) du véhicule (100) est rejeté si le segment est plus court qu'une valeur de seuil de durée prédéfinie.

9. Procédé (400) selon l'une des revendications précédentes, **caractérisé en ce qu'**à l'étape de détermination (430), le premier et/ou deuxième signal d'ouverture d'oeil (245, 250) reconnu comme étant valide et/ou le signal d'ouverture d'oeil global est lissé.

10. Procédé (400) selon l'une des revendications précédentes, **caractérisé en ce qu'**à l'étape de reconnaissance (420), le premier et/ou deuxième signal d'ouverture d'oeil (210, 220) et/ou le premier et/ou deuxième signal d'ouverture d'oeil (245, 250) reconnu comme étant valide représente un degré d'ouverture d'oeil qui est plus grand qu'une valeur de seuil de degré d'ouverture d'oeil prédéterminée.

11. Dispositif (110), lequel est réalisé pour exécuter, mettre en oeuvre et/ou commander toutes les étapes d'un procédé (400) selon l'une des revendications précédentes dans des unités (215, 230, 240) correspondantes.

12. Programme informatique étudié pour exécuter, mettre en oeuvre et/ou commander toutes les étapes d'un procédé (400) selon l'une des revendications 1 à 10 précédentes.

13. Support d'enregistrement lisible par ordinateur avec un programme informatique selon la revendication 12 enregistré sur celui-ci.
